# EUROPEAN PATENT APPLICATION

(11) **EP 1 321 176 A1**
(43) Date of publication of application: **25.06.2003**
(21) Application number: 01130067.0
(22) Date of filing: 18.12.2001
(51) Int. Cl.: B01D 15/08, C07H 1/08, C12N 15/10

(54) **Method and device for isolating and purifying a polynucleotide of interest on a manufacturing scale**

(71) Applicant: Boehringer Ingelheim International GmbH, 55216 Ingelheim (DE); Bia Separations D.O.O., 1111 Ljubljana (SI)
(72) Inventor: Necina, Roman, 1210 Wien (AT); Urthaler, Joehen, 2344 Maria Enzersdorf (AT); Podgornik, Ales, 1000 Ljubljana (SI); Strancar, Ales, 5270 Ajdovscina (SI); Jancar, Jancz, 1230 Domzale (SI); Merhar, Mojca, 1331 Dolenja vas (SI); Barut, Milos, 1000 Ljubljana (SI)
(74) Representative: Laudien, Dieter, Dr.

(57) **Abstract**

A process for isolating and purifying a polynucleotide on a manufacturing scale uses a chromatographic separation process, which comprises a hydrophobic interaction chromatography step and an anion exchange chromatography step. In at least one of the two steps the chromatographic support is a porous monolithic bed. Chromatographic device and its use for isolating and purifying a polynucleotide of interest, in particular plasmid DNA, on manufacturing scale.

## Description

The present invention pertains to a method and device for isolating and purifying a polynucleotide of interest on a manufacturing scale.

The developments in molecular and cell biology in the last quarter of the 20^{th} century have led to new technologies for the production of complex biomolecules, which are increasingly used in human health care in the areas of diagnostics, prevention and treatment of diseases. At first, research was mainly directed to protein molecules, while lately some of the most revolutionary advances have been made with polynucleotides in the field of gene therapy and nucleic acid vaccines.

Representative members of polynucleotides are messenger RNA (mRNA), genomic DNA (gDNA), and plasmid DNA (pDNA). Polynucleotides are a heterogeneous group of molecules in terms of size, shape, physico-chemical properties and biological function. Common to all of them are their building blocks (A,G,C,T,U) and their high negative charge under physiological conditions. Polynucleotides can be single- or double-stranded. Similarly to proteins, they are able to build structures and multimers. Some species, e.g. mRNA, are very labile in their natural environment, in cell lysates or even in purified form, partly due to the presence of DNases and RNases. Due to their size gDNA and pDNA are very sensitive to mechanical and shear forces. Therefore, polynucleotide-containing solutions need to be handled very carefully and gently.

One of the most important and at the same time most expensive steps in the production of polynucleotides is their isolation and purification (down-stream processing). More than 50% of the total costs of the production process are incurred by these operations. Precipitation, extraction, ultrafiltration, and liquid chromatographic techniques are most widely used for these purposes. Typically, these methods are combined with each other. Liquid chromatography is the most powerful method, which allows manufacturing of a product suited for therapeutic use.

During the last few years, gene therapy has become a promising therapeutic approach in human medicine. DNA plasmid-based treatment is considered an alternative to classical chemical drugs or proteins recovered from recombinant cells. Potential applications of plasmid DNA (pDNA) are in the treatment of acquired and genetic disease and in vaccines. The plasmid carries information that allows expression of a protein of interest in the targeted human cells; in addition it contains regulatory elements that control expression in the host cell. pDNA is formulated for ex vivo or in vivo administration, jointly with viral or non-viral gene delivery vehicles.

Due to the increasing amounts of pDNA required for preclinical and clinical trials, there is a demand for processes to be performed on a manufacturing scale. These production processes must fulfil regulatory requirements (FDA, EMEA) and should be economically feasible.

At present, *E.coli* is the most commonly used production host, but other bacterial hosts, yeasts, mammalian and insect cells may also be used as host cells. A high copy number per cell and stable maintenance during the fermentation are crucial for a robust process with high yield. Fermentation is performed in batch or fed-batch mode. Since fed batch processes reach higher cell densities (OD > 100) they are considered to be superior for large-scale production. After fermentation the cell broth is harvested by centrifugation, aliquoted, and frozen. For downstream processing of pDNA cells are thawn and broken up. A combination of column chromatography and/or precipitation steps is utilized for purification of pDNA. After a final sterile filtration, the pDNA bulk is aliquoted and stored under proper conditions.

In manufacturing processes, the requirements for pDNA are different from those for recombinant proteins, because the two types of macromolecules differ significantly in their physico-chemical properties. Plasmids are negatively charged over a wide pH range, are large and have a long, thin shape. A typical plasmid contains between 5 and 20 kilo base pairs which corresponds to 3 x 10⁶-13 x 10⁶ Da and several thousand Å. Its shape is responsible for its sensitivity against mechanical stress.

There are several different forms of pDNA. The supercoiled or covalently closed circular (ccc) form is the most stable form. The degree of supercoiling is dependent on the environmental conditions, such as temperature and pH. The open circular (oc) (or nicked) form is produced by breaking a single strand. Breakage of both strands can be caused by chemical and physical stress and produces the linear form. Since ccc pDNA is considered the most potent and stable form of pDNA, the final product obtained from the production process should contain more than 90% of this form. The amount of ccc pDNA is considered as one of the most important characteristics for the quality of the pDNA preparation.

Critical points in manufacturing scale production of plasmid DNA are cell lysis, subsequent clarification of the cell lysate, purification by a combination of different techniques, such as chromatography, extraction and precipitation.

Plasmid DNA, due to its size and shape, is very sensitive to shear forces. Therefore, lysis of cells must not be performed by using a high-pressure technique such as homogenization. Chemical and enzymatic e.g. using (lysozyme) methods cause minimal mechanical stress and minimal irreversible changes of the plasmid. During cell lysis under alkaline conditions, cells are subjected to NaOH and SDS. Subsequent neutralization to pH 5.5 causes flocculation of cell debris, proteins, and gDNA. Very often RNAse is added to digest RNA into small pieces in order not to not interfere during the downstream process. After addition of NaOH and SDS, the solution becomes highly viscous. Mixing without destroying the plasmid is difficult. Usually glass bottles containing the viscous solution are mixed very gently by hand. Some processes use optimized tanks and stirrers or a combination of different mixers in order to overcome these problems.

The isolation and purification of large polynucleotides like plasmid DNA is hampered by the low performance of commercially available chromatographic sorbents, which are mainly based on highly porous particles. Most of the chromatographic supports were tailor made for the high adsorption capacity of proteins with particle pore diameter of typically 30-400 nm (protein diameter typically <5 nm). In these processes, large polynucleotide molecules like pDNA (with a size of 30 to >300 nm in diameter) adsorb only at the particle surface, leading to capacities ca. 50 times lower than those achieved with proteins. The design and application of new chromatographic supports and devices that would allow high capacity polynucleotide purification is therefore crucial to move gene therapy forward.

As a rule, liquid chromatography is a rather slow process. It often causes significant product degradation and requires expensive separation media, large volumes of solvents, a long process time and high investments for buffer tanks and other chromatography-related equipment. Diffusional constraints within the large pores of the porous particle in particular limit the speed of separation, especially of larger molecules, as they cause a rapid reduction in resolution with increasing elution velocity in the case of conventionally packed columns. On the other hand, the efficient isolation of labile, valuable biomolecules requires a fast, reliable and affordable separation process under mild conditions.

Most processes available for pDNA purification are conducted on a small laboratory scale. They mainly involve cell lysis using enzymes like RNAse or lysozyme, extraction with organic solvents and ultracentrifugation in density gradients. Most of these processes are time consuming and not scalable. Furthermore, due to the usage of material that is not certified for application in humans, the usage of enzymes and toxic reagents such as phenol, CsCl, CsBr, etc., these processes are not suitable to meet approval by regulatory authorities.

In the last few years there have been several attempts to develop manufacturing scale processes for pDNA purification. US-A-5,981,735 describes a process for large-scale pDNA purification based on anion exchange and gel permeation chromatography. For this process, the use of RNAse during the cell lysis is suggested. The first chromatography step represents an anion exchange expanded or fluidized bed packed with porous particles (capacities 0,05 - 0,1 mg pDNA/ml). To produce a pharmaceutical grade plasmid DNA a second purification step is needed using high-resolution anion exchange column packed with porous particles with optional use of Triton X-100 or Tween 20 to remove impurities. Final polishing is achieved by gel permeation chromatography.

US-A-6,197,553 describes a process for large-scale plasmid purification using two chromatographic steps, i.e. an anion exchange chromatography as the first step and reversed phase chromatography as the second step. Major drawbacks are, besides using porous particles in both chromatography steps, usage of RNAse and, during the cell lysis, usage of lysozyme. In addition, toxic organic solvents are required for the second chromatographic step.

US-A-6,242,220 discloses a process for purification of ccc pDNA using only one chromatography step, i.e. an anion exchange column. Besides using porous particle chromatographic column the drawback is the use of nuclease to cleave the gDNA, oc DNA and linear DNA.

The established processes are of limited usefulness due to various drawbacks:

All these processes are based on chromatography using various types of porous particles optimized for protein purification and exhibit low or very low capacities for pDNA, furthermore, they are slow in the separation process. Therefore, large chromatographic columns are necessary for purification of polynucleotides in gram scale. For equilibration, washing and elution large buffer volumes are necessary. Some of the resins are for single-use only and need to be replaced after each run. Column emptying, cleaning and packing need to be taken into account for every run. This has a significant input on the process time, costs of goods and the size of equipment such as tanks for buffer and wastewater treatment. Limited separation capability of the applied chromatographic resins requires a combination with extraction or precipitation steps. As these processes require usage of organic, toxic solvents several safety issues need to be addressed; these also need to be taken into account for most of the detergents. Several established processes require the usage of animal-derived enzymes. Usage of flammable, organic solvents requires explosion-proofed production areas, which contribute to a major part to the production costs. In addition, precautions for the safety of patients, operators and the protection of the environment need to be implemented.

An alternative to using porous particles is the use of membrane technology, which reflects technological advances in both membrane filtration (ultrafiltration) and fixed-bed liquid chromatography (Heath CA, Belfort G (1992) Adv Biochem Eng/Biotechnol 47:45; Zeng XF, Ruckenstein E (1999) Biotechnol Progr 15:1003). Ultrafiltration membranes (filters) are employed mainly as »cut off« devices; they can separate biomolecules whose sizes differ by one order of magnitude or more. When affinity, ion exchange, hydrophobic interaction or reversed phase ligands are coupled to such membranes (filters), an increase in selectivity can be achieved. The chromatographic interactions in the membrane are usually similar to those in the porous particulate material. They mainly differ in their hydrodynamic properties.
Membrane-based chromatography can generally be distinguished from porous particle-based chromatography in that the interaction between a solute (for example proteins or pDNA) and a matrix (immobilized ligand) does not take place in the dead-end pores of a porous particle, but mainly in the flow-through pores of the membrane. While the mass transport in dead-ended pores necessarily takes place by diffusion, the liquid moves through the pores of the membrane by convective flow, dramatically reducing the long diffusion time required by conventional particle-based chromatography. As a consequence, membrane separation processes are generally very fast, in fact at least one order of magnitude faster than columns packed with the corresponding porous particles. In addition, most of the active sites are exposed at the surface of the flow-through channels, which can result in higher capacities for big molecules.

Since membranes are very thin beds (usually a few mm stack of several thin sheets with large diameter) as compared to chromatographic columns packed with porous particles, reduced pressure drop is found along the chromatographic unit, allowing increased flow rates and consequently higher productivity ((Heath CA, Belfort G (1992) Adv Biochem Eng/Biotechnol 47:45; Zeng XF, Ruckenstein E (1999) Biotechnol Progr 15:1003).

Many membrane separations are performed by using a conventional filtration apparatus; others are configured for compatibility with existing chromatography pumps and detectors. Regardless of the configuration of the apparatus and the type of matrix, the problem of uniform flow distribution from a relatively thin pipe to a large area has to be solved, as well as the problem of recollecting the eluate at the other end of the device with minimal back-mixing and distortion of zones, to improve the resolution power of the membrane device. Another problem lies in their relatively large dead volume within the unit resulting in large band spreading which in consequence lowers the resolution power of the membrane chromatography units.

Apart from a predominantly diffusive transport, the problem of particulate separation media is their inability to completely fill the space within the chromatographic column, the latter problem being valid for membranes as well. This results in peak broadening and decreased column efficiency.

An even higher degree of continuity than with membranes was achieved with the introduction of monoliths.

A monolith is a continuous bed consisting of a single piece of a highly porous solid material where the void volume is decreased to a minimum (Tennikova TB, Svec F (1993) J Chromatogr 646:279). The most important feature of this medium is that all the mobile phase is forced to flow through the large pores of the medium. As a consequence, mass transport is enhanced by convection and has a positive effect on the separation. Three types of monolithic supports are currently commercially available
- Silica gel based monolithic beds: These columns are solid rods of silica monolith that have been prepared according to a sol-gel process. This process is based on the hydrolysis and polycondensation of alkoxysilanes in the presence of watersoluble polymers. The method leads to "rods" made of a single piece of porous silica with a defined bimodal pore structure having macro (of about 2 µm) and mesopores (of about 0,013 µm) when smaller rods intended for analytical purposes are prepared. The main feature of these columns is about 80% porosity, which is 15% more than columns packed with standard particulate packing. As a result, the pressure drop along the column is one-third to one-fifth of that on columns packed with 3 µm or 5 µm beads. Scale-up columns, which are suitable for laboratory and semi-industrial purification have macro pores of about 4 µm and mesopores of about 0,014 µm and this allows even higher flow rates to be used then in case of the analytical ones (Nakanishi K, Soga N (1991) J Am Ceram Soc 74:2518; Cabrera K, Wieland G, Lubda D, Nakanishi K, Soga N, Minakuchi H, Unger KK (1998) Trends Anal Chem 17:50).
- Polyacrylamide based monolithic beds are made of swollen polyacrylamide gel compressed in the shape of columns. Their technology relies on the polymerization of advanced monomers and ionomers directly in the chromatographic column. In the presence of salt, the polymer chains form aggregates into large bundles by hydrophobic interaction, creating voids between the bundles (irregularly shaped channels) large enough to permit a high hydrodynamic flow. Following polymerization, the bed is compressed by connecting it to an HPLC pump adjusted to a flow rate equal or higher than that used in subsequent runs. The obtained bed can be regarded as a rod or plug permeated by channels in which the eluent can pass upon application of pressure. The polymer chains form a dense, homogeneous network of nodules consisting of microparticles with an average diameter of 2 µm. The channels between the nodules are large enough to permit a high hydrodynamic flow beds (Hjerten S, Liao J-L, Zhang R (1989) J Chromatogr 473:273; Liao J-L, Zhang R, Hjerten S (1991) J Chromatogr 586:21).
- Rigid organic gel based monolithic beds: These supports are prepared by free radical polymerization of a mixture of a polymerizable monomer, optionally with functional groups, such as glycidyl methacrylate, ethylene dimethacrylate, a crosslinking agent, a radical chain initiator, such as 2,2'-azobisisobutyronitrile, and porogenic solvents (cyclohexanol and dodecanol) in barrels of an appropriate mold (Svec F, Tennikova TB (1991) J Bioact Compat Polym 6:393; Svec F, Jelinkova M, Votavova E (1991) Angew Macromol Chem 188:167; Svec F, Frechet JMJ (1992) Anal Chem 64:820) in the case of glycidyl methacrylate-co-ethylene dimethacrylate (GMA-EDMA) monoliths. Another method uses free radical polymerization of a mixture of styrene and divinylbenzene (as a cross-linking reagent) using 2,2'-azobisisobutyronitrile as an initiator and porogenic solvents (dodecanol and toluene) (Merhar M, Podgornik A, Barut M, Jak 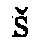 a S, 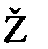 igon M, 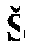 trancar A (2001) J Liq Chromatogr 24:2429). After polymerization, the formed block of polymer is washed, e.g. with methanol, followed by a methanol-water mixture (50:50) and distilled water to remove porogenes and residual monomers from the polymer. After this the monolithic bed is ready for derivatization to achieve a desired chemistry or immobilization of ligands. GMA-EDMA monoliths have active epoxide groups which can easily be further modified using various chemicals e.g. diethyl amine, propane sulfone for ion exchange chromatography, e.g. butyl groups for hydrophobic interaction chromatography and any desired protein ligand for affinity chromatography.

In adsorptive chromatographic modes the slope of the capacity factor k', defined as the molar ratio of the separated compound in the stationary phase and the mobile phase, plot versus the composition of the mobile phase is very steep. Up to a certain composition of the mobile phase, the k' value may become so high that the protein is bound to the stationary phase only and does not move along the column. Reaching a defined point, a small change of the mobile phase composition causes a rapid decrease in k' to a value near zero. At this point, the protein dissolves in the mobile phase and passes through the column practically without any retention. In other words, the macromolecule remains adsorbed at the top of the column until the eluting power of the mobile phase reaches the point at which a small change in the composition of the mobile phase causes the movement of the protein without any retention. One can also speak about selective elution of the compound. As a result of this process, even very short columns can provide very good separations and very good recovery, while longer columns might cause problems due to unspecific binding, product degradation and minor changes in the structure of the biomolecule which increase with the length of the column (Tennikova TB, Belenkii BG, Svec F (1990) J Liq Chromatogr 13:63; Strancar A, Barut M, Podgornik A, Koselj P, Josic D, Buchacher A (1998) LC-GC Int 10:660).

On the basis of this postulation, new types of monolithic columns were developed in the shape of disks and, for the scale-up applications, in the shape of tubes (WO-A-96/06158; US-A-5,972,218; EP-A-777725) and so-called "tube in a tube"devices as disclosed in (WO-A-99/44053). The disclosure of these references is incorporated by reference. "Monolithic columns" are available under the trade name CIM® disk monolithic columns and CIM® tube monolithic columns.

Scale up of particle columns containing particles that range in size from a few micrometers up to 100 micrometers can be achieved by packing of these small particles in larger columns. In contrast thereto, large-scale monolithic columns are obtained by producing a large block of a polymer casted in a proper cartridge (monolith holder). An issue to be addressed is preparing such a monolith with a uniform structure. In the following, this process will be elucidated, using as an example methacrylate monoliths prepared by polymerization.

When the polymerization temperature is reached, the initiator decomposes and oligomer nuclei start to form. The solubility of the polymers in the reaction mixture decreases during the growth phase and they start to precipitate. In terms of their thermodynamic properties, the monomers are better solvating agents for the polymer than the porogenes. Consequently, the precipitated nuclei are swollen in the presence of the monomers. Since the monomer concentration is higher than in the surrounding solution, polymerization in the nuclei is kinetically preferred. In the absence of mixing, due to higher density, insoluble nuclei sediment and accumulate at the bottom of the mould. Initially, they form a very loose structure, which is highly porous. In the course of polymerization, nuclei continue to grow and crosslink until the final structure is achieved. As can be deduced from the above description, the pore size distribution of the polymer depends on its chemical composition and the polymerization temperature. Namely, the temperature defines the degradation rate of the initiator and, therefore, also the number of nuclei formed in a given time. Since the amount of the monomers is constant, the lower number of nuclei formed at lower temperatures within a defined volume corresponds to their larger size, thus, to larger pores between the clusters of growing nuclei. In contrast, at higher polymerization temperatures, at which the decomposition of initiator occurs much faster, the number of growing nuclei is much larger and, as a consequence, the formed pores are much smaller. Therefore, the polymerization temperature is a powerful tool for the control of pore formation.

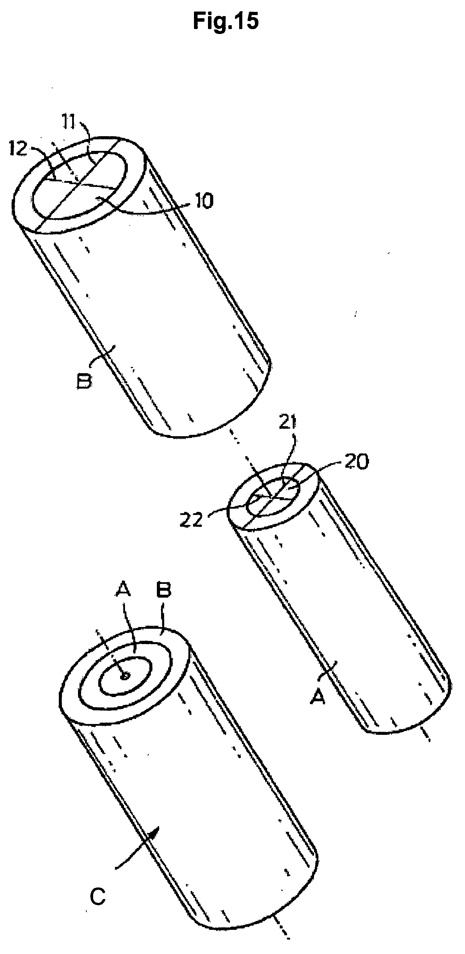

Polymerization of a methacrylate-based monolith is an exothermic process. Therefore, heat is released during the reaction. With no mixing being employed and with a size of the mould in a range of centimeters, the released heat cannot dissipate fast enough. As a consequence, an increase of the temperature inside the reaction mixture occurs. In a mould of e.g. 50 mm, a temperature increase in the range of 100°C was observed and a 25°C temperature differential was recorded across the radius of the column. Pore size distribution measurements revealed that the pores in the middle of the polymer are larger than on the outer part, resulting in an inhomogeneous pore size distribution. Obviously; the preparation of large volume monoliths is limited due to the exothermic nature of the polymerization and due to a pronounced temperature influence on the pore size distribution. To overcome these problems, the so-called "tube in a tube" design, as disclosed in WO-A-99/44053, which is herewith incorporated by reference, was developed. Instead of gradually adding the polymerized mixture to form a single large volume monolith, this approach is based on the construction of a monolithic annulus of a required radius but limited thickness. However, since it is possible to construct the annuluses where the outer diameter of a smaller monolith is equal to the inner diameter of a larger one, a large volume monolithic unit can be constructed by inserting two or several annuluses one into another as shown in Fig. 15, forming a so-called "tube in a tube" system. In this way, a monolithic unit of required volume and uniform pore size distribution can be obtained. Furthermore, the voids between the annuluses can be filled with the reaction mixture and polymerization is allowed to proceed for a second time. Since the voids are very thin, no increase in temperature during the course of the reaction is expected.

This approach was at first verified by the construction of an 80 ml "tube in a tube" monolithic column. The monolithic column was characterized by low backpressures even at a high flow rates (below 2.5 MPa at the flow rate of 250 ml/min). One interesting feature is that in contrast to conventional radial columns of large diameter and a small bed thickness, the bed in this case had an outer diameter that was 35 mm while the inner diameter was only 1.5 mm. Due to this, the linear velocity of the mobile phase increases more then 23 times from the outer to the inner surface of the column. In the case of conventional porous particle supports, such changes in the linear velocity would generally result in a pronounced deterioration of the column efficiency. However, the characteristics of the monoliths were found to be flow independent, therefore the change in linear velocity should not have any influence either on the resolution or on the binding capacity. This was proved by the separation of a protein mixture as well as by measuring the dynamic binding capacity determined at different flow-rates. This concept was further verified by construction and characterization of the 800 ml monolithic column.

Plasmid DNA separation using disc monolithic columns was first described by Giovannini et al (Giovannini R, Freitag R, Tennikova TB (1998) Anal Chem 70:3348). The authors reported that by using optimized conditions a pure pDNA sample can be separated into 3 peaks which presumably correspond to supercoiled (ccc), open circular and linearized pDNA. Separations under gradient and isocratic conditions were studied and it was shown that different forms of pDNA could be separated on a strong anion exchange unit by, in contrast to protein chromatography, using isocratic conditions. The results using disk monolithic columns were compared with those obtained from similar experiments using a conventional column packed with 10µm porous particles and monolithic type column based on soft gel (UNO, BioRad). The analysis reported here was performed with a purified pDNA sample.

The potential of disk monolithic columns for the separation of nanoparticles (pDNA and measles virus) was investigated by Branovic et al. (Branovic K, Forcic D, Santak M, Kosutic-Gulija T, Zgorelec R, Mazuran R, Trescec A, Benko B (2000), Poster P 023 presented at the 20th International Symposium on the Separation and Analysis of Proteins, Peptides, and Polynucleotides - ISPPP 2000, Ljubljana, Slovenia). The authors successfully separated pDNA from cell RNA. Furthermore, they successfully used the DEAE disk monolithic columns for a very large molecular structure, i.e. for purification and concentration of measles virus.

It was an object of the invention to provide a manufacturing scale method for purifying polynucleotides, in particular plasmid DNA, for therapeutic applications, that overcomes the limitations and shortcomings of the available methods. In particular, it was sought to provide a process that can be applied to pDNA of 100 kbp and above, that results in a high degree of purity of plasmid DNA in the ccc form and a low content of host RNA, proteins, chromosomal DNA and endotoxins. Furthermore, it was desirable that the process should neither require the use of enzymes like RNAse and lysozyme nor the use of detergents, organic solvents and expensive and unsafe chemicals like CsBr, CsCl, LiCl.

The solution of the problem underlying the invention is based on the selection of specific chromatographic principles and the use of separation media with a higher degree of continuity than conventional media.

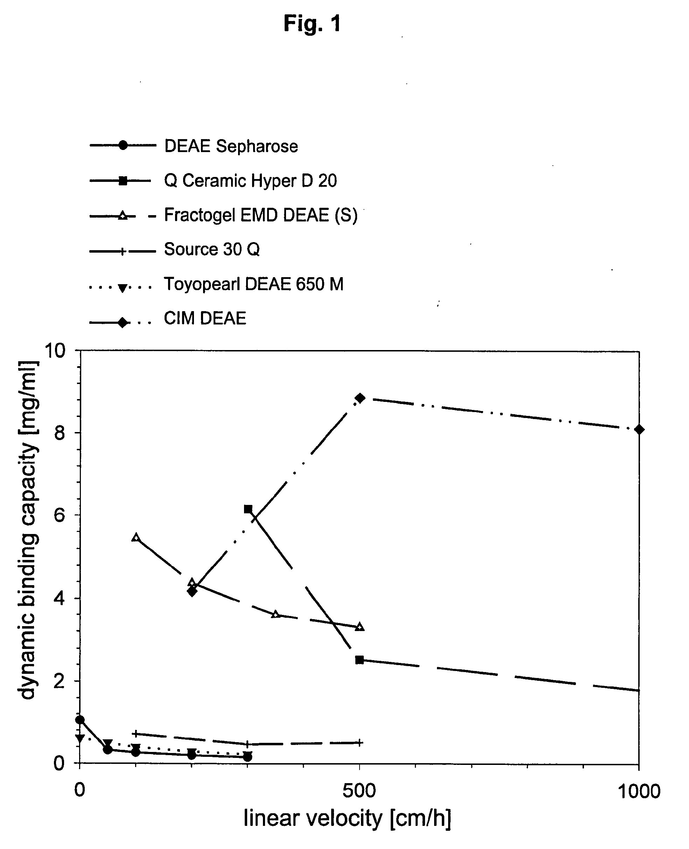

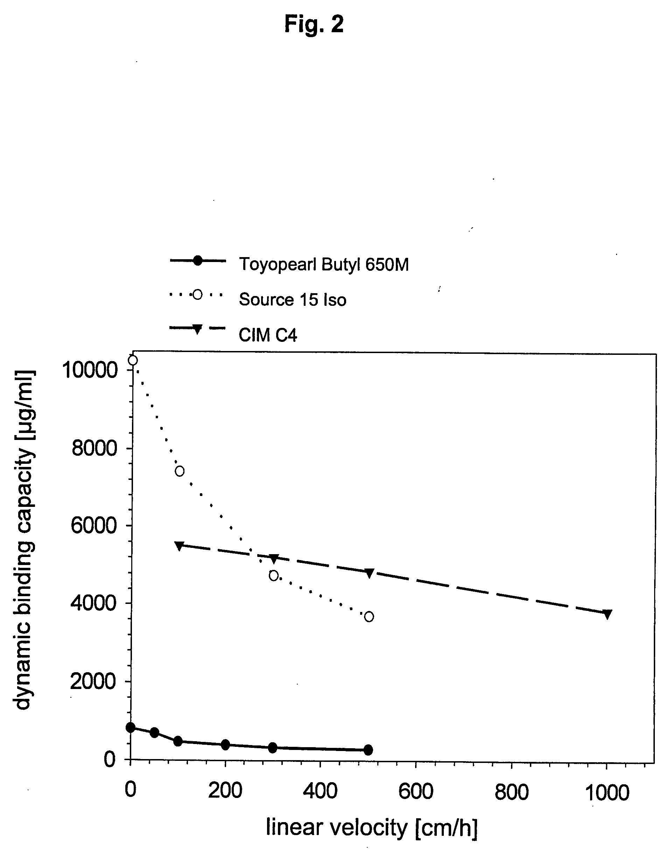

The above-discussed references show the potential of a methacrylate-based monolithic supports for separation of large molecules, while they do not demonstrate pDNA-binding capacity. The capacity of the methacrylate-based monoliths is typically in the range of 20-30 mg per ml of supports for proteins, while the capacity of some particulate supports reaches values between 100 and 200 mg of protein per ml of support. In preliminary tests of the present invention, it was surprisingly found that the capacity of methacrylate-based monoliths ranges from several mg to above 10 mg pDNA per ml of support material while typical values for particulate supports are in the range of 1 mg per ml. This is especially true for a dynamic binding capacity, which remains high with the increase of the linear velocity for the monoliths, while decreases significantly in the case of particulate supports. This comparison is presented in Figs. 1 and 2. The high dynamic binding capacity of the support is advantageous because it significantly increases the productivity of a purification process. Furthermore, due to shorter purification steps, any degradation of the target pDNA molecules is reduced, resulting in higher yield.

The present invention relates to a process for isolating and purifying a polynucleotide of interest on a manufacturing scale, wherein a preparation containing said polynucleotide of interest is subjected to a chromatographic separation process. The process is characterized in that
a) the chromatographic separation process comprises a hydrophobic interaction chromatography step and an anion exchange chromatography step; and in that
b) in at least one of the two steps the chromatographic support is a porous monolithic bed.

With regard to the polynucleotide of interest, the applicability of the process is not limited or restricted by its the size or its function. The polynucleotide of interest may be a DNA or RNA molecule with a size range of 0.1 to approximately 100 kb and above. Preferably, the polynucleotide of interest is circular DNA, i.e. plasmid DNA with a size of preferably 1 to 20 kb. For linear DNA or RNA molecules, the process parameters that have been designed for plasmid DNA may be adapted by introducing other ligands, changing the ligand density, changing binding and elution conditions or even the sequence of the purification steps.

Also, the process of the invention is not limited with regard to the source from which the polynucleotide of interest has been obtained. Usually, the preparation containing the polynucleotide of interest is obtained from a biological source. In particular, the preparation is a cell lysate, in most cases a bacterial lysate, containing, as the polynucleotide of interest, plasmid DNA. The cell lysate is obtained from the biomass, according to known methods (e.g. alkaline lysis), from a fermentation process in which host cells, usually bacteria, are cultivated to produce the plasmid. While the *E.coli* cells are the most commonly used host cells in plasmid DNA production, the cell lysate may also have been obtained from other bacterial hosts, yeasts, mammalian and insect cells.

The process of the invention is also independent of the source from which the polynucleotide of interest has to be isolated. Plasmids or other polynucleotides that are enclosed in cells or cellular compartments, respectively, are made accessible by breakage of the cellular structures either by chemical, enzymatic or mechanical methods or combinations thereof. The composition of the resulting preparation has no restricting influence on the usage of this invented process.

In the meaning of the present invention, a "manufacturing scale" process is defined by being capable of processing large volumes of a polynucleotide containing preparation, typically more than 20 liter, and yielding amounts of the polynucleotide of interest that meet the demands for clinical trials as well as for market supply, i.e. amounts ranging from 0.1g to several 100 g.

In the meaning of the present invention, a monolith (or monolithic bed) is a continuous bed consisting of a single piece of a highly porous solid material where the pores are highly interconnected forming a network of flow-through channels. The monolith skeleton can be made of organic or inorganic material, examples for monolith materials are silica, acrylamide, methacrylate. The skeleton is functionalized with the chemical moieties that are able to interact with the polynucleotide molecules via various interaction modes, i.e. ion exchange or hydrophobic interactions.

In the process of the present invention, any monolithic support can be used which is permeable for the polynucleotide of interest, preferably for plasmid DNA. To this end, the monolith advantageously has a porosity defined by wide channels with a diameter of > 1 µm and narrow channels with a diameter of ≤ 100 nm.

The monolith preferably has a dynamic binding capacity of >8 mg/ml at average linear velocities of up to 1000 cm/h for polynucleotides smaller than 10 kbp.

As described above, the monolith bed carries functional moieties (ligands) that allow for the specific chromatographic separation. The ligand density is chosen such that capacity, yield and recovery are maximized.

In view of producing pDNA for application in gene therapy, the monolithic material preferably fulfils the requirement that it can be sterilized (either thermally or chemically).

Furthermore, the monolithic material resists the cleaning procedures of the column, e.g. high pH values. (Usually cleaning of the column is achieved by treatment with 0.5 - 1M NaOH for several hours. For increased efficiency, warm (e.g. 50°C) NaOH solution can be used.)

Rigid methacrylate-based monoliths as described above (Tennikova TB, Belenkii BG, Svec F (1990) J Liq Chromatogr 13:63; Strancar A, Barut M, Podgornik A, Koselj P, Josic D, Buchacher A (1998) LC-GC Int 10:660; WO-A-96/06158; WO99/44053; Svec F, Tennikova TB (1991) J Bioact Compat Polym 6:393; Svec F, Jelinkova M, Votavova E (1991) Angew Macromol Chem 188:167; Svec F, Frechet JMJ (1992) Anal Chem 64:820), which fulfil the above requirements, are preferred in the present invention for manufacturing scale production of pharmaceutical-grade biopolynucleotides.

Preferably, methacrylate-based monolith columns in the form of tubes, as described in WO-A-96/0615; US-A-5,972,218; EP-A-777725 and WO-A-99/44053, are used in the present invention. Such tubes are commercially available from BIA Separations, Slovenia, as CIM® (Connective Interaction Media) tubes.

In the case the hydrophobic interaction chromatography (HIC) step is the one using a monolithic support, the monolith carries functional moieties (ligands) suitable for hydrophobic interaction, e.g. ethyl groups or butyl groups. The principles of HIC, including the selection of suitable ligands and ligand density, that maybe applied when using a monolithic support, are known in the art and can be found in e.g. in Pharmacia Biotech: Hydrophobic Interaction Chromatography: Principles and methods, Västra Aros Tryckeri AB, Västerås, 1993 and in the references described herein.

In this case, the anion exchange chromatography step (A-IEX) may be carried out on a conventional chromatographic support carrying functional moieties suitable for anion exchange, e.g. DEAE (Diethylaminoethyl) or QA (quaternary ammonium) groups (Himmelhoch, SR(1971) Chromatography of proteins on ion-exchange adsorbents. Meth. Enzymol. 22: 273 -286).

In the case the A-IEX step uses a monolithic support, the monolith carries functional moieties (ligands) suitable for anion exchange, e.g. DEAE or QA groups. The principles of A-IEX, including the selection of suitable ligands and ligand density, that may be applied when using a monolithic support, are known in the art and can be found in e.g. Pharmacia Pharmacia Biotech: Ion Exchange Chromatography:
Principles and methods, Västra Aros Tryckeri AB, Västerås) and in the references described herein.

In this case, HIC is may be carried out on a conventional chromatographic support carrying functional moieties (ligands) suitable for hydrophobic interaction, e.g. ethyl-, butyl-groups (Hofstee BHJ(1973) Hydrophobic affinity chromatography of proteins. Anal. Biochem. 52: 430 - 448).

In a preferred embodiment, both the HIC and the A-IEX step use a monolithic support. For this embodiment, the process parameters are the same as described above for the embodiments that employ the monolithic support in only one of the two steps.

In a preferred embodiment, hydrophobic interaction chromatography (HIC) is carried out as the first step and the anion exchange chromatography step (A-IEX) is carried out as the second step.

The parameters of the process of the invention are advantageously designed such that no manipulation or adjustment of the buffer conditions (conductivity, pH) of the pool obtained from the first chromatographic step is necessary for further processing by the second chromatographic step. Thus, the pool from the first step can be directly used for second step. By way of example, if the first chromatographic step is HIC and the second chromatographic step is A-IEX, for both steps buffers containing Tris and EDTA, adjusted to the same pH, are used. Elution from the first column is achieved by decreasing the salt concentration (conductivity) in the mobile phase. The eluate pool from the first step is directly used for the second chromatographic step without further processing. In the second chromatographic step elution is achieved by increasing the salt concentration.

In another preferred embodiment, the HIC step and the A-IEX step are carried out on a single monolithic column, which is represented by the "tube in a tube", design described above (WO 99/44053). In this design, the outer and inner tube carry different functional moieties, one of the monolithic tubes representing the chromatographic support for HIC and the other one for A-IEX.

The present invention also relates to a chromatographic device suitable for carrying out the process of the invention. Atypical device is shown in Fig. 15.

In the case of using the "tube in a tube" monolith, usually, since the preparation flows from the periphery to the center of the device, the outer tube (or annulus) represents the porous monolithic support in the first step and the inner tube functions as the porous monolithic support in the second step. Preferably, the outer tube is equipped with functional groups for HIC and the inner tube is equipped with functional groups for A-IEX. The chromatographic device of the invention is a chromatographic device as disclosed in principle in WO-A-99/44053, which device having a porous self-supporting structure comprising at least two porous components A and B and the porous component B embraces the porous component A, wherein surfaces of pores of the at least two porous components A and B are provided with functional moieties suitable for chromatography of substances passing the pores and the pores of the porous components comprise a uniform multimodal pore size distribution through the entire polymeric structure. The device of the invention is characterized in that
(i) the porous surfaces of component A are modified with functional moieties suitable for hydrophobic interaction chromatography (HIC) and
(ii) the porous surfaces of component B are modified with functional moieties suitable for anion exchange chromatography (A-IEX).

Fig. 15 shows an assembly C, the porous polymer tube, of two different components, component A and component B. The two components can be inserted into each other to form a multi monolith porous polymer tube, wherein component A forms the inner part, and component B the outer part of the concentric assembly C. Component B comprises an inner lumen 10 which diameter 12 is large enough to match the outer diameter 21 of component A. Component A finally comprises a central bore extending through the entire length of component C. The central bore works as sample collector in the assembly C of Fig. 15. If the central bore has larger diameter an element can be inserted to minimize the dead volume of the collector as well as to provide additional mechanical stability.

According to the invention, component A has been modified with functional moieties, which are suitable for A-IEX. Functional moieties (ligands) suitable for anion exchange are e.g. DEAE, EDA or QA (quaternary ammonium) groups. The methods for modifying the surfaces of component A with these ligands are well known, e. g. Pharmacia Biotech: Ion Exchange Chromatography: Principles and methods, Västra Aros Tryckeri AB, Västerås) and in the references described herein.

According to the invention, component B has been modified with functional moieties, which are suitable for HIC. Functional moieties (ligands) suitable for hydrophobic interaction are e.g. ethyl, butyl, phenyl groups. The methods for modifying the surfaces of component B with these ligands are well known, e. g. Pharmacia Biotech:
Hydrophobic Interaction Chromatography: Principles and methods, Vastra Aros Tryckeri AB, Västerås, 1993 and in the references described herein.

It is also possible to add further components, which carry ligands suitable for performing other chromatographic steps.

Subject of the present invention is also the use of a chromatographic device having a porous self-supporting structure comprising at least two porous components A and B and the porous component B embraces the porous component A, for isolating and purifying a polynucleotide of interest, in particular plasmid DNA as defined above, on a manufacturing scale. The surfaces of pores of the at least two porous components A and B are provided with functional moieties suitable for chromatography of substances passing the pores and the pores of the porous components comprise a uniform multimodal pore size distribution through the entire polymeric structure.
- The porous surfaces of component A are modified with functional moieties suitable for hydrophobic interaction chromatography (HIC) or anion exchange chromatography (A-IEX) and
- the porous surfaces of component B are modified with functional moieties suitable for anion exchange chromatography (A-IEX) or hydrophobic interaction chromatography (HIC) whereby
- the porous surfaces of components A and B are not simultaneously modified with the moieties of (i) or (ii).

Depending on the requirements of the specific final product, for adjustment of the final concentration of the polynucleotide molecule of interest, a third purification step, a so-called "polishing step", may be conducted after the HIC and the A-IEX step, e.g. to obtain a change to a buffer that is suitable for the formulation of the pDNA product, and the removal of remaining impurities. This polishing step may be any further chromatographic step, preferably gel permeation chromatography or an ultrafiltration step, carried out according to standard methodology; preferably, the polishing step may also be carried out on a porous monolithic support.

Thus, in a preferred embodiment, the method of the invention comprises three steps, wherein the final polishing step is a gel permeation chromatography step (preferally carried out on a monolithic support) or an ultrafiltration step.

During the first and the second step (and optionally during the third step) the preparation containing the polynucleotide of interest is eluted from the column. Elution is achieved by standard methods, e.g. decreasing (HIC) or increasing (A-IEX) salt concentration (conductivity, ionic strength) or change in pH-value of the mobile phase (Pharmacia Biotech: Hydrophobic Interaction Chromatography:
Principles and methods, Västra Aros Tryckeri AB, Västerås, 1993; Pharmacia Biotech: Ion Exchange Chromatography: Principles and methods, Västra Aros Tryckeri AB, Västerås). In the case of using the "tube in a tube" system, elution is carried out by elution from the HIC section by decreasing ionic strength of the mobile phase and by further increase of the mobile phase's ionic strength for elution from the A-IEX section of the column.

The process can be operated in batch-wise mode, quasi-continuous or continuous mode. As batch-wise operation is limited in terms of productivity, the process of the invention can be even more advantageous when operated in a quasi-continuous or continuous mode. Technologies such as annular chromatography, carousel chromatography and simulated moving bed are compatible with the process of the invention, monolithic material can be used in any of these systems.

The process of the invention features an outstanding productivity (speed and capacity) and, when used for plasmid DNA, a high degree of purity concurrently with a high yield of ccc pDNA (according to conventional methods, a high purity can usually only be achieved at the expense of yield). In the process of the invention, apart from high yield, a homogeneity of more than 95% ccc pDNA of the total pDNA can be achieved (even 99% ccc pDNA is possible). The final product meets all regulatory requirements.

Furthermore, the process of the invention has the following advantages:
- it allows purification of pDNA of the size of more than 100 kbp;
- it is scalable for isolating several grams to several 100 g amounts of pDNA in a single batch;
- it does not require any enzymes like RNAse and lysozyme;
- it does not require chemicals like CsBr, CsCl, LiCl;
- it does not require toxic detergents;
- it does not require organic solvents;
- it does not require any precipitation or extraction step;
- it uses chromatographic supports and devices that allow cleaning-in-place (CIP) and sterilization-in-place (SIP) procedures;
- the number of cycles of the used chromatographic monolithic supports is typically more than 50.

Due to these advantages, the process and device of the invention are suitable for cGMP (Current Good Manufacturing Practice) production of pharmaceutical grade pDNA. The process can be adapted to any source of pDNA. In particular due to the properties of the monolithic columns, the process of the invention allows fast processing of large volumes, which is of major importance for processing cell lysates containing only low concentrations of pDNA, typically about 100 µg/m. Since the lysates contain various pDNA-degrading substances such as DNAses, process speed is a key to high product quality and yield.

The process and device of the invention are suited for production of pDNA for use in humans and animals, e.g. for vaccination and gene therapy applications. Due to its high productivity, the process can be used for production of preclinical and clinical material as well as for market supply of a registered product.

### Examples

### Preliminary experiments:

### Determination of dynamic binding capacity

Several chromatographic supports for anion exchange chromatography and hydrophic interaction chromatographie were testet to determine their dynamic binding capacity for pDNA. This was carried out by recording break-through curves and calculating the bound amount of pDNA per ml resin at 10% break-through. Each material was tested at different linear velocities.

Before use, the resins, packed into a column with a volume of 1 to 2ml, were washed with 0.5M NaOH and subsequently equilibrated with loading buffer. The loading buffer for the A-IEX resins contained 0.1M Tris, 0.01M EDTA and 0.3M NaCl at pH 7. The loading buffer for the HIC resins contained 0.1M Tris, 0.01M EDTA and 3M ammonium sulfate. As load a stock-solution (1mg/ml) of a purified 6.9kbp pDNA was diluted with the corresponding loading buffer. This pDNA solution was loaded onto the particular chromatographic support till break-through (UV absorbance) stayed constant. The loaded amount of pDNA at 10% of the break-through UV absorbance indicated the dynamic binding capacity at the particular linear velocity for the tested resin. Fig. 1 shows the comparison of the dynamic binding capacity at 10% breakthrough of the tested anion exchange chromatography supports at different linear velocities. Fig. 2 shows the comparison of the dynamic binding capacity at 10% breakthrough of the tested hydrophobic interaction chromatography supports at different linear velocities.

All examples were performed with pDNA containing *E. coli* biomass produced as follows.

### Preculture

The working cell bank of the production strain of the plasmid pRZ-hMCP1 (*Escherichia coli* K12 JM108; ATTC no. 47107; plasmid size: 4892 kbp) was maintained in cryo vials (glycerol stocks) at -70°C. A cryo vial of the working cell bank was defrosted at room temperature for 15 min and a 200 µL aliquot thereof was inoculated in a 1000 mL Erlenmayer shake flask containing 200 mL autoclaved preculture medium (composition in gL⁻¹: Vegetable Peptone/Oxoid 13.5; Bacto Yeast Extract/Difco 7.0; glycerol 15.0; NaCl 1.25; MgSO₄*7H₂O 0.25; K₂HPO₄2.3; KH₂PO₄1.5). The preculture was incubated at 37° ± 0.5°C and 150 rpm up to an optical density (OD₅₅₀) of 1-1.5.

### Fermenter Preparation

A fermenter of a total volume of 30 L (continuous stirred tank reactor) was used for fermentation. Three of the medium components (in gL⁻¹ final culture medium: Vegetable Peptone/Oxoid 13.5; Bacto Yeast Extract/Difco 7; glycerol 15) was heat sterilized inside the fermenter at 121 °C for 20 min. After cooling down of the fermenter content to ≤ 40°C, a macro element solution (in gL⁻¹ final culture medium: tri-Sodium citrate dihydrate 0.5; KH₂PO₄ 1.2; (NH₄)₂SO₄ 5.0; MgSO₄*7H₂O 8.8; Na₂HPO₄*12H₂O 2.2; CaCl₂*2H₂O 0.26; NH₄Cl 4.0) was sterile filtered into the fermenter. By sterile filtration with a syringe, 5 mL of a 1 % m/v thiamin solution and 1.5 mL of a trace element solution was transferred into the fermenter. The trace element solution consists of (in gL⁻¹ solution): CoCl₂*6H₂O 0.9; CuSO₄*5H₂O 1.23; FeSO₄*7H₂O 38.17; MnSO₄*H₂O 1.82; Na2MoO₄*2H₂O 0.48; NiSO₄*6H₂O 0.12, ZnSO₄*7H₂O 5.14. The fermentation medium was filled up with sterile deionized water to the final working volume of 20 L.

### Fermentation and Harvest of Cell Paste

The total preculture volume of 200 mL was transferred into the fermenter under sterile conditions. The cultivation conditions were set as follows: aeration rate 20 Lmin⁻¹ = 1 vvm; agitation rate 400 rpm, 37 ± 0.5 °C; 0.5 bar; pH 7.0 ± 0.2). The pH r automatically controlled with 5 M NaOH and 25 % m/v H₂SO₄. The dissolved oxygen tension (DO, pO₂) was maintained at ≥ 20 % by automatic agitation rate control (400-700 rpm).

The cultivation was terminated 12 h after the inoculation of the fermenter. After cooling down to ≤ 10 °C, the culture broth was harvested and then separated in an ice water-cooled tube centrifuge. The obtained cell paste was packaged and stored at-70°C.

The frozen cells were thawn and disrupted by alcaline lysis based on the protocol of Birnboim and Doly, (1979) Nucleic Acid Res Nov. 24;7(6):1513-23. Subsequently the lysate was clarified by a combination of centrifugation and filtration.

Chromatography: Chromatographic supports were packed into XK columns from Amersham Pharmacia Biotech or Moduline from Millipore.

CiM tubes were provided by BIA Separations.

Chemicals were purchased from Merck.

### Example 1

### Purification of pDNA by hydrophobic interaction chromatography (HIC) and anion exchange chromatography (A-IEX) using monolithic tubes in both steps

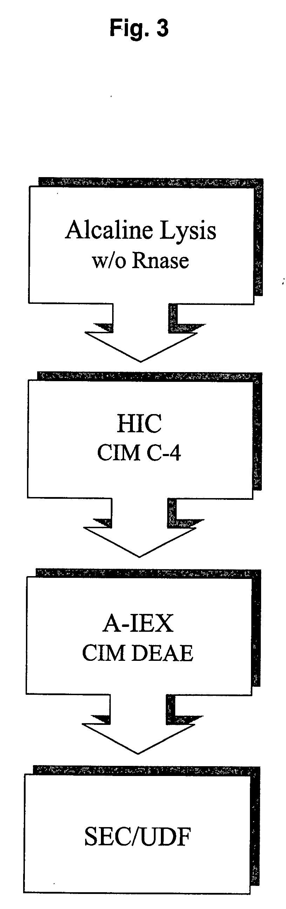

Purification of pDNA from a crude E. coli cell lysate was achieved by a combination of a 80ml (i.d.: 3.5cm, height: 8.5cm) CIM-C4 (methacrylate based monolithic support with butyl ligands) tube (HIC), 80ml (i.d.: 3.5cm, height: 8.5cm) and a CIM DEAF (methacrylate-based monolithic support with diethylaminoethyl ligands) tube (A-IEX) and a final gel permeation chromatograpy (size exclusion, SEC) step (column: i.d. = 9cm, height = 80cm). Fig. 3 shows a flow chart of the pDNA purification process. The downstream part consists of two chromatographic steps utilizing CIM tubes. For the final polishing step, alternatively to SEC, as used in this experiment, ultrafiltration (UF) can be used.

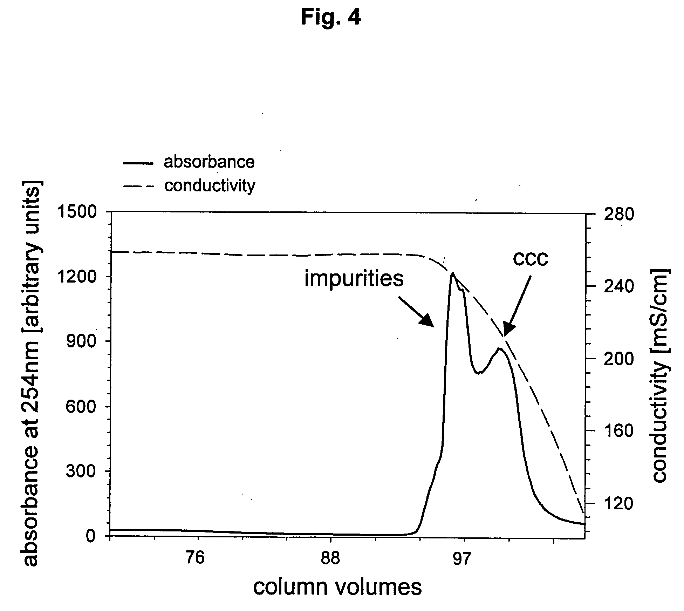

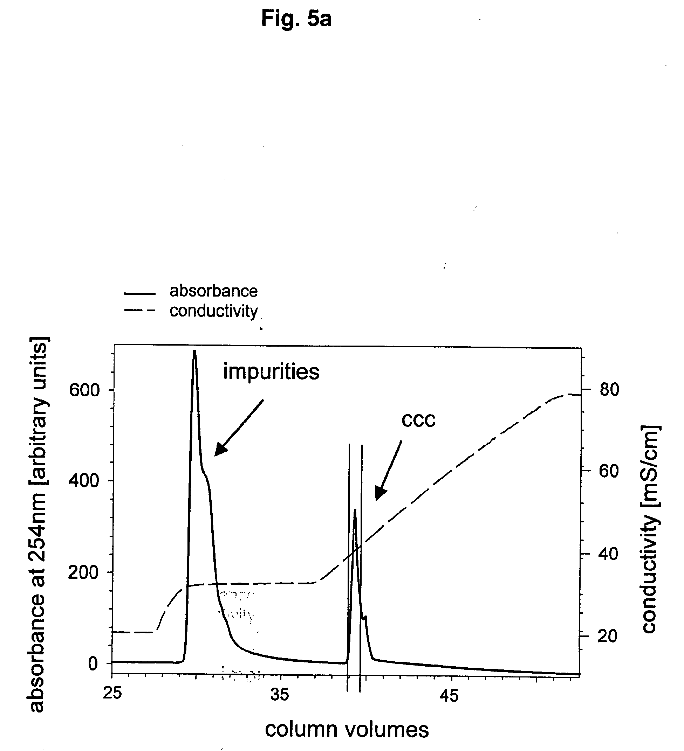

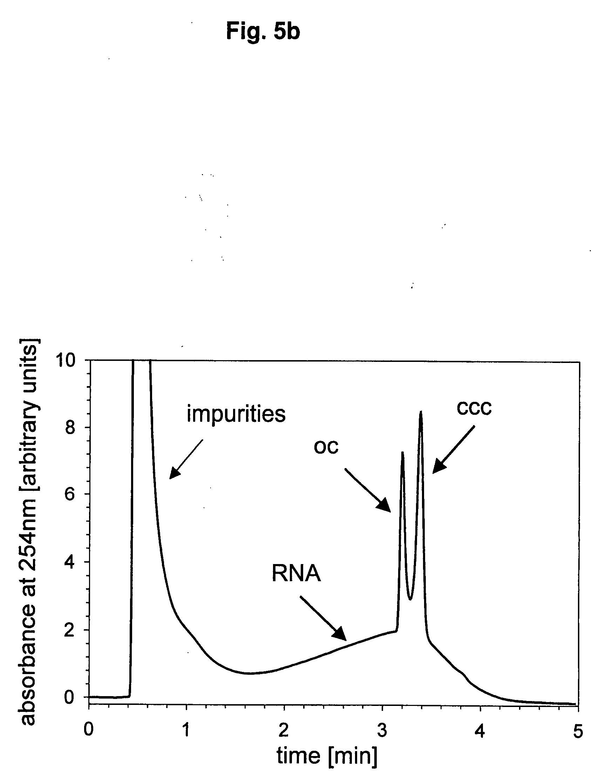

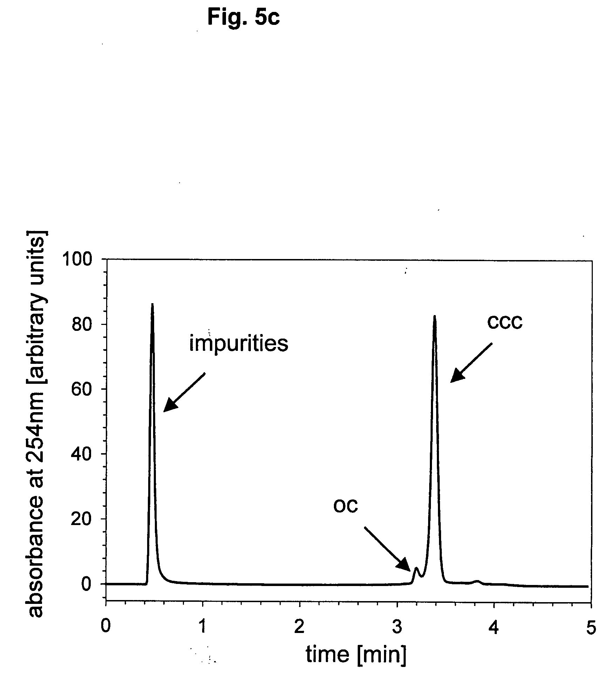

The clarified lysate was adjusted to 3M ammonium sulfate (AS). After an additional clarification step by filtration the solution was loaded onto the equilibrated CIM C-4 tube. After finishing the load step, the column was washed thoroughly with the equilibration buffer (0.1M Tris, 0.01M EDTA, 3M AS, pH 7.0). By decreasing the AS concentration to 0 M, bound material was eluted (see Fig. 4). The first peak contained RNA, chromosomal DNA and oc pDNA whereas the second peak contained mainly ccc pDNA. Fractions were analysed by IEX HPLC and ccc pDNA containing fractions were loaded onto the equlibrated (0.1M Tris, 0.01 M EDTA, 0.5 M NaCl pH 7.0) CIM DEAF tube. After washing the tube with 5 cv of wash buffer (0.1M Tris, 0.01 M EDTA, 0.3 M NaCl pH 7.0) bound pDNA was eluted by an increasing salt gradient to 1.5 M NaCl (see Fig. 5a). The large peak obtained as eluate during washing contained mainly chromosomal DNA, RNA and a small amount of oc and ccc pDNA (see HPLC analysis in Fig. 5b). During elution with an increasing salt gradient highly purified ccc pDNA eluted in a concentrated form (see HPLC analysis in Fig. 5c). Fractions were collected, analysed and pooled according to their ccc pDNA content. The pDNA pool was split into two portions.

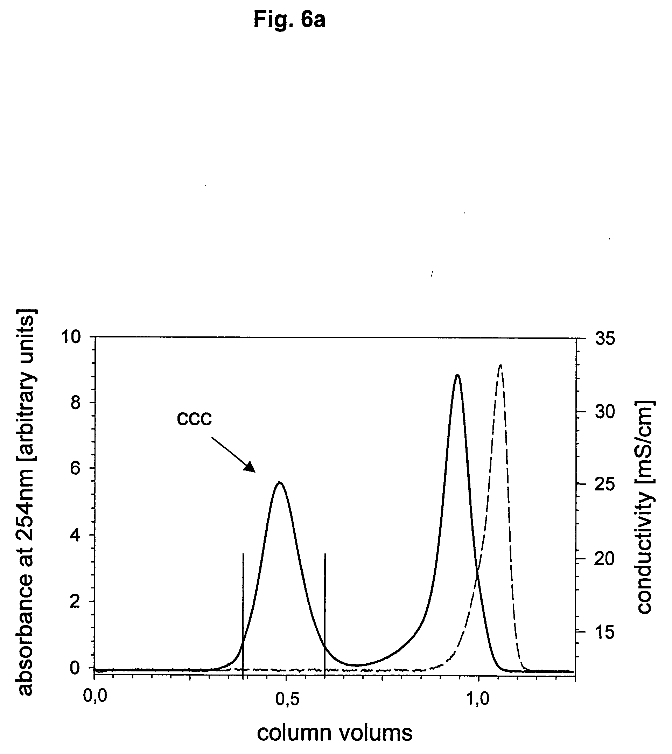

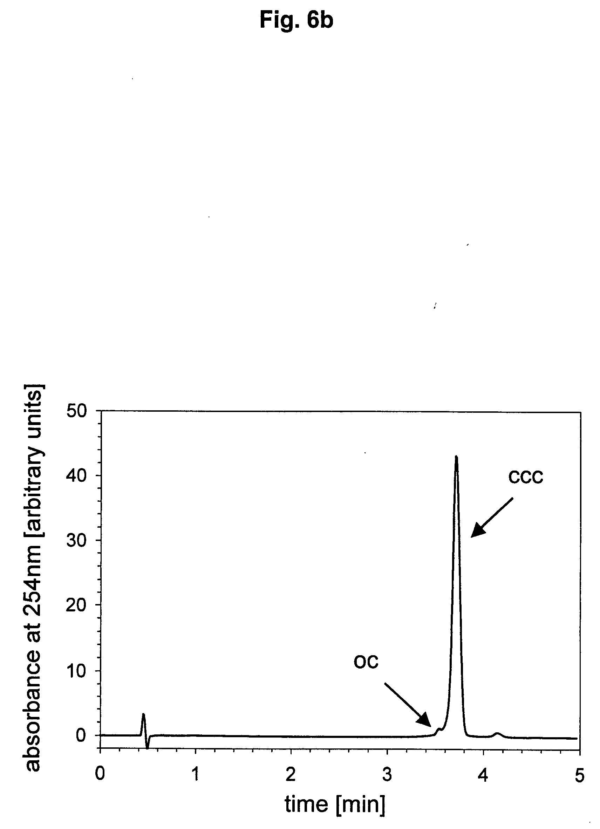

Half of the A-IEX pool was loaded onto an equilibrated Sephacryl S-1000 SF (Amersham Biosciences, Sweden) column. The running buffer consisted of PBS pH 7.4.. This purification step is shown in Fig. 6a). As evident from the HPLC analysis shown in Fig. 6b the pooled fractions of the first peak contained more than 95 % ccc DNA.) The pDNA containing peak was collected and 0,2 µm filtrated.

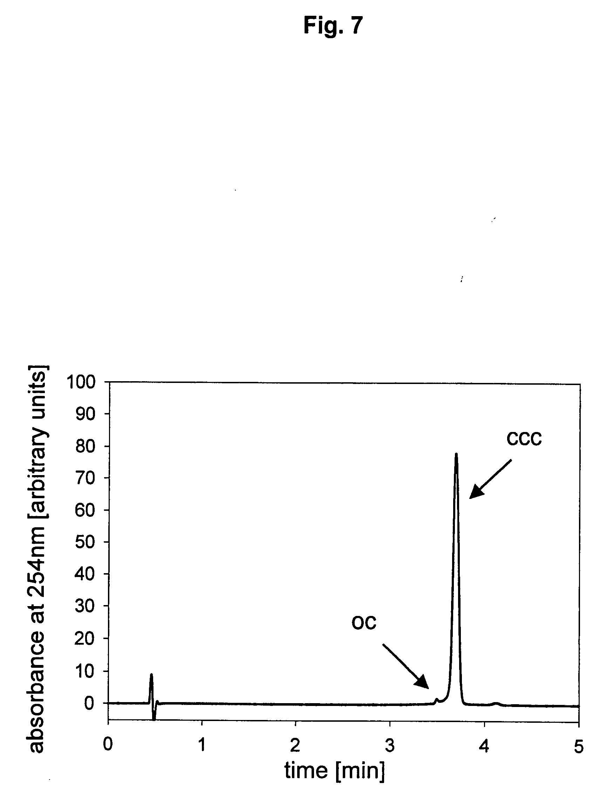

The second half of the sample was subjected to ultrafiltration. The A-IEX pool was ultrafiltrated utilizing a regenerated cellulose membrane with a MWC of 50 kDa. After ten volumes buffer exchange to PBS pH 7.4, the retentate was 0.2 µm filtrated and analysed. Analysis by analytical HPLC (see Fig. 7) shows that the retentate contained highly purified ccc pDNA).

### Example 2

### Purification of pDNA by hydrophobic interaction chromatography (HIC) and anion exchange chromatography (A-IEX) using a monolithic tube in the HIC step.

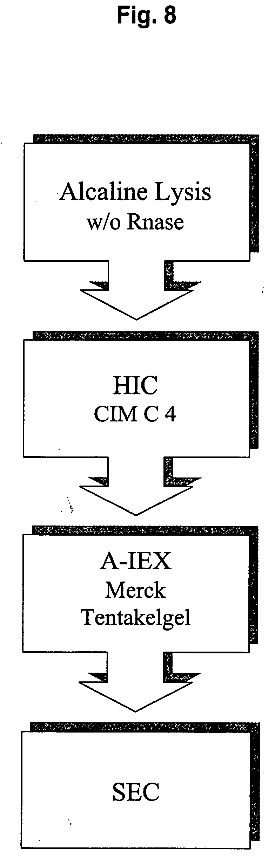

The purification pathway for isolation of pDNA from a crude cell lysate combined the CIM C-4 tube (HIC) with a non monolithic A-IEX material such as the Fractogel EMD DEAE (Merck, Germany) and a final Size Exclusion Chromatography (see the flow chart in Fig. 8). The ammonium sulfate-adjusted and clarified lysate was loaded onto a 80ml (i.d.: 3.5cm, height: 8.5cm) CIM-C4 (methacrylate based monolithic support with butyl ligands) tube as in Example 1. The HIC pool was further processed by A-IEX (Fractogel EMD DEAE)with a column of 2.6cm i.d. and a bed height of 20cm. Final polishing was achieved by a size exclusion chromatography (SEC) step (column: i.d. = 9cm, height = 80cm).

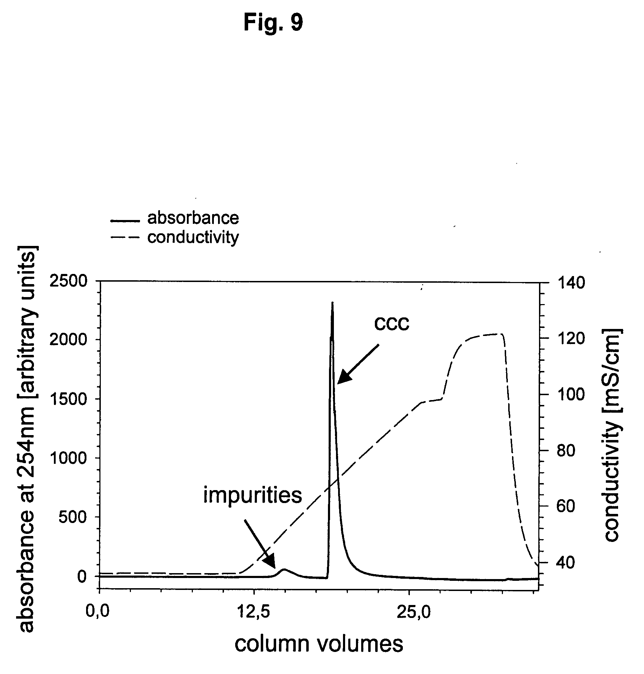

The clarified lysate was adjusted to 3 M ammonium sulfate and clarified by filtration. Subsequently the solution was loaded onto the equilibrated CIM C-4 tube. Washing and euilibration was obtained with a 0.1 M Tris buffer pH 7.0 containing 0.01M EDTA and 3M AS. By decreasing the AS concentration to 0 M, bound material was eluted. ccc pDNA containing fractions were pooled and loaded onto the equlibrated (0.1M Tris, 0.01 M EDTA, 0.5 M NaCl pH 7.0) Fractogel EMD DEAF column. After washing with wash buffer (0.1M Tris, 0.01M EDTA, 0.3 M NaCl pH 7.0) elution could be obtained with an increasing salt gradient (see Fig. 9: highly purified ccc pDNA eluted in concentrated form). The A-IEX pool was further processed by SEC (or alternatively UF).

### Example 3

### Purification of pDNA by hydrophobic interaction chromatography (HIC) and anion exchange chromatography (A-IEX) using a monolithic tube in the A-IEX step

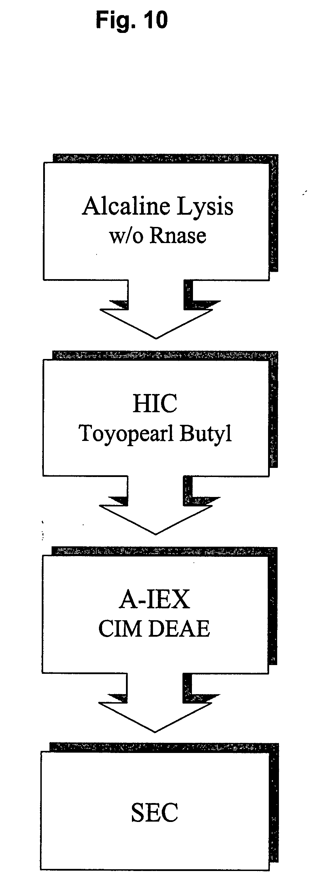

In this Example, isolation of pDNA from crude E. coli lysate was achieved by combining a non-monolithic chromatographic material, in this case the Toyopearl Butyl 650M (Tosoh, Japan), packed into a column of an i.d. of 9cm and a bed height of 19cm, with the 80ml (i.d.: 3.5cm, height: 8.5cm) CIM DEAF (methacrylate based monolithic support with diethylaminoethyl ligands) tube and a final size exclusion chromatography (column: i.d. = 9cm, height = 80cm) (or ultrafiltration step) as shown in the flow chart in Fig. 10.

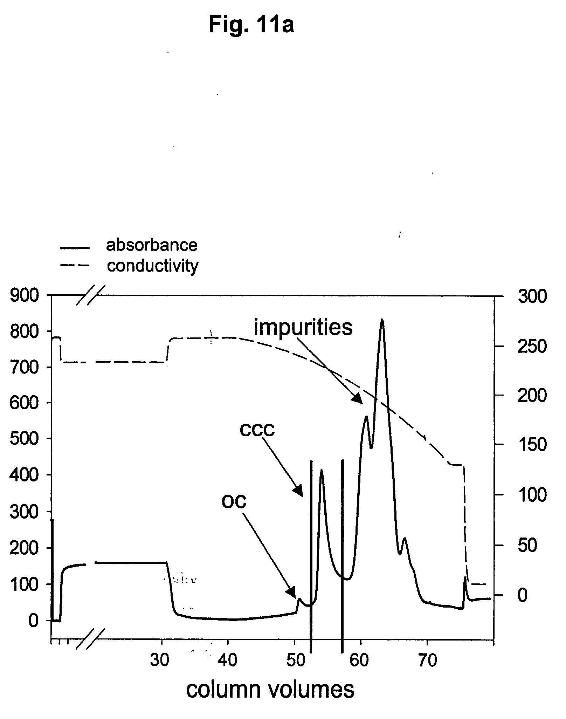

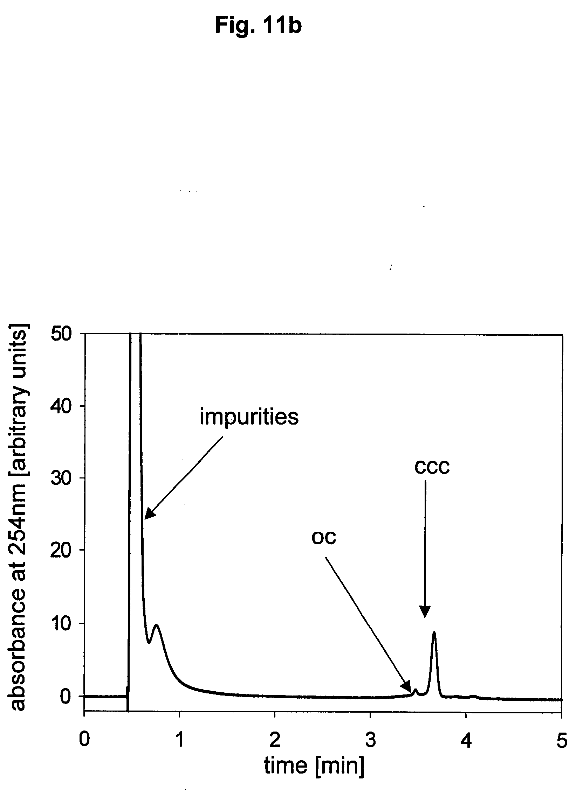

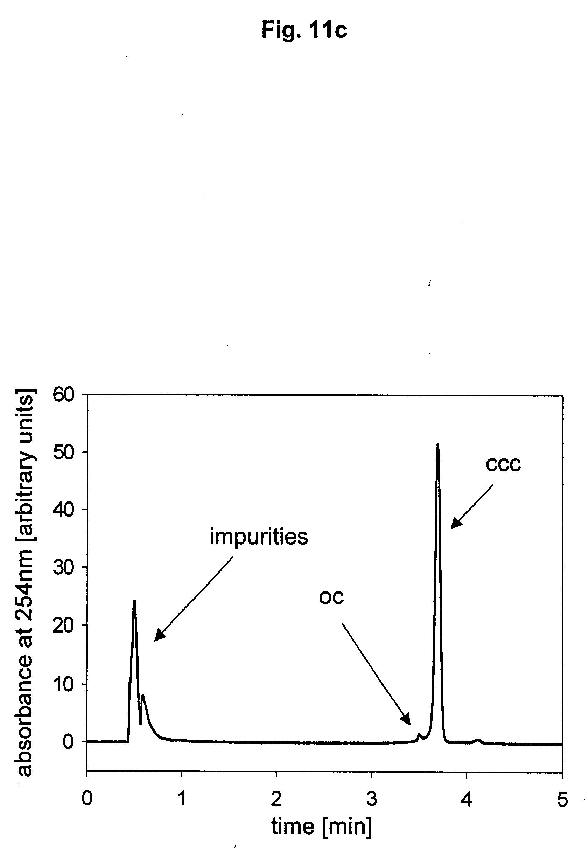

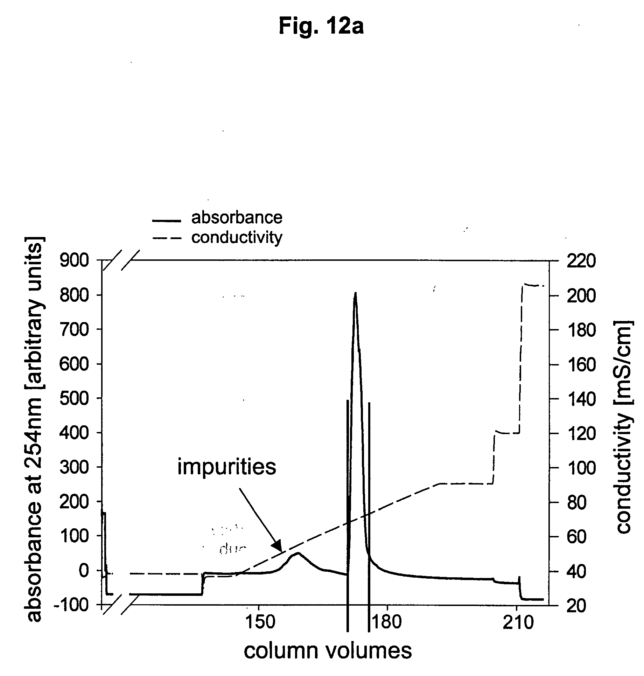

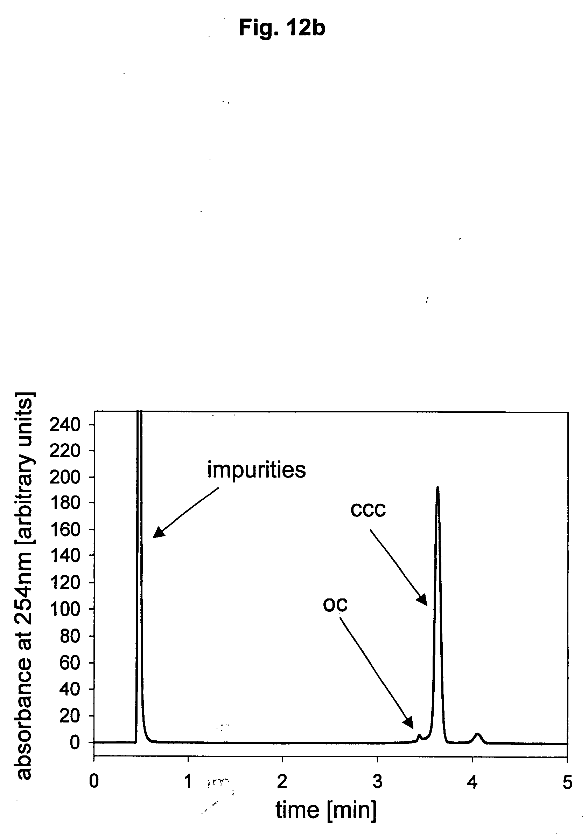

After clarifying the lysate and adjusting it to 3M AS the Toyopearl Butyl 650 M column was loaded. Subsequently the column was washed with 5 cv of the equilibration buffer (0.1M Tris pH 7, 0.01M EDTA, 3M AS). Elution of the bound material was obtained by a decreasing AS gradient (see Fig. 11a). The load (Fig. 11b) and the eluted fractions were analysed by HPLC. The ccc pDNA fractions were pooled (HPLC analysis shown in Fig. 11c) and loaded onto the CIM DEAF tube which was equilibrated with 0.1 M Tris buffer pH 7 containing 0.01M EDTA and 0.3 M NaCl. After washing, bound material was eluted with an increasing NaCl gradient to 1.5 M NaCl (see Fig. 12a). The HPLC analysis of the pooled fractions (second peak) is shown in Fig. 12b. Further processing of the A-IEX pool was performed with SEC (or optionally UF), as already described.

### Example 4

### Purification of pDNA by anion exchange chromatography (A-IEX) and hydrophobic interaction chromatography (HIC) using a monolithic tube in the A-IEX step

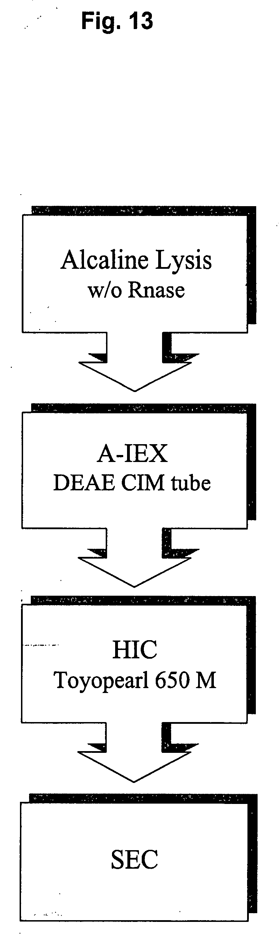

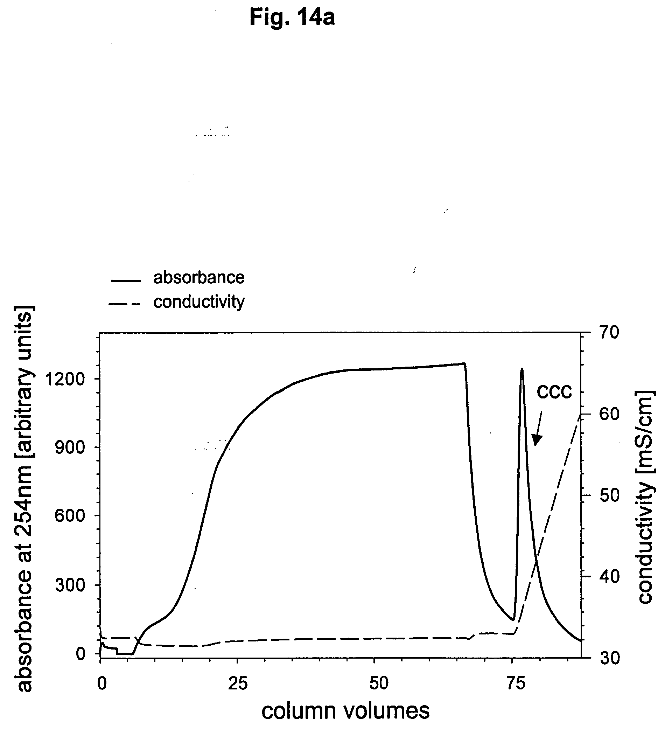

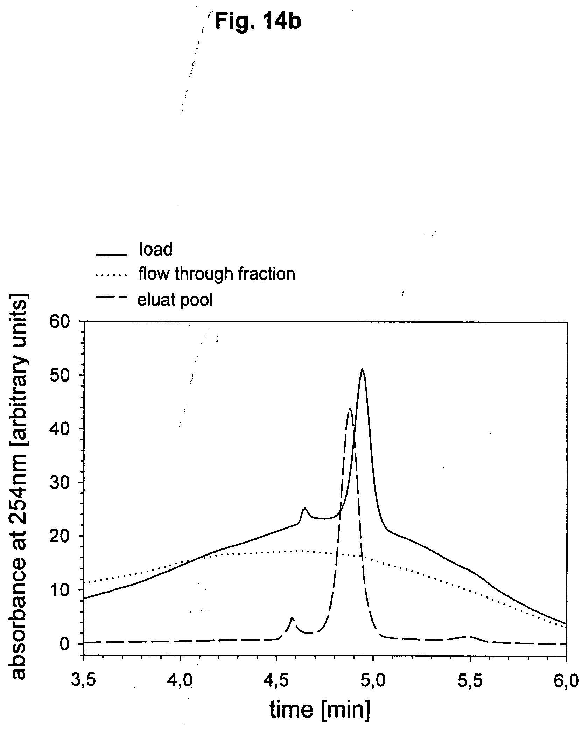

In these experiments, a combination of the 80ml (i.d.: 3.5cm, height: 8.5cm) CIM DEAE (methacrylate based monolithic support with diethylaminoethyl ligands) tube with non-monolithic HIC, in this case the Toyopearl Butyl 650M (Tosoh, Japan), packed into a column of an i.d. of 9cm and a bed height of 19cm, was used for isolation of pDNA from an E. coli lysate (see the flow chart in Fig. 13).The clarified lysate was loaded onto the CIM DEAF tube,which was equilibrated with 0.1M Tris buffer pH 7 containing 0.01 M EDTA and 0.3M NaCl. After washing bound material was eluted with an increasing NaCl gradient to 1.5 M NaCl (see Fig. 14a). Fig. 14b shows the HPLC analysis of the load, the flow-through fraction and the peak fraction. In the peak fraction the ccc pDNA could be determined.

Further processing was performed with a Toyopearl Butyl 650M column and SEC or UF as described in the previous examples.

## Claims

1. A process for isolating and purifying a polynucleotide of interest on a manufacturing scale, wherein a preparation containing said polynucleotide of interest is subjected to a chromatographic separation process, **characterized in that**
a) the chromatographic separation process comprises a hydrophobic interaction chromatography step and an anion exchange chromatography step; and **in that**
b) in at least one of the two steps the chromatographic support is a porous monolithic bed.

2. The process of claim 1, wherein said polynucleotide of interest is plasmid DNA.

3. The process of claim 1 or 2, wherein said preparation is derived from a biological source.

4. The process of claim 3, wherein said preparation is a cell lysate.

5. The process of claim 4, wherein said preparation is a bacterial lysate.

6. The process of claim 1, wherein hydrophobic interaction chromatography is carried out as the first step and anion exchange chromatography is carried out as the second step.

7. The process of claim 1, wherein said monolithic bed is a rigid methacrylate-based monolith.

8. The process of claim 2 and 7, wherein the monolithic bed has a porosity defined by wide channels with a diameter of ≥ 1µm and narrow channels with a diameter of ≤ 100nm.

9. The process of claim 1, wherein the monolithic bed has a dynamic binding capacity of >8 mg/ml at average linear velocities of ≤ 1000 cm/h for polynucleotides smaller than 10 kbp.

10. The process of claim 1, wherein the monolithic bed is in the form of a tube.

11. The process of claim 1, wherein the chromatographic support is a monolithic bed in both the hydrophobic interaction chromatography step and the anion exchange chromatography step.

12. The process of claim 11, wherein the chromatographic support is a single monolithic bed comprising a tube in a tube, the outer and inner tube carrying different functional moieties, whereby one of the monolithic tubes represents the support for hydrophobic interaction chromatography and the other one for anion exchange chromatography.

13. The process of claim 12, wherein the outer tube represents the support for hydrophobic interaction chromatography.

14. The process of claim 1, wherein the process additionally comprises a final purification step.

15. The process of claim 14, wherein the final purification step is a chromatography step.

16. The process of claim 15, wherein the final purification step is gel permeation chromatography.

17. The process of claim 15 or 16, wherein the chromatographic support is a porous monolithic bed.

18. The process of claim 14, wherein the final purification step is an ultrafiltration step.

19. A chromatographic device having a porous self-supporting structure comprising at least two porous components A and B and the porous component B embraces the porous component A, wherein surfaces of pores of the at least two porous components A and B are provided with functional moieties suitable for chromatography of substances passing the pores and the pores of the porous components comprise a uniform multimodal pore size distribution through the entire polymeric structure, **characterized in that**
(i) the porous surfaces of component A are modified with functional moieties suitable for hydrophobic interaction chromatography (HIC) and
(ii) the porous surfaces of component B are modified with functional moieties suitable for anion exchange chromatography (A-IEX).

20. Use of a chromatographic device having a porous self-supporting structure comprising at least two porous components A and B and the porous component B embraces the porous component A, wherein surfaces of pores of the at least two porous components A and B are provided with functional moieties suitable for chromatography of substances passing the pores and the pores of the porous components comprise a uniform multimodal pore size distribution through the entire polymeric structure, **characterized in that**
(i) the porous surfaces of component A are modified with functional moieties suitable for hydrophobic interaction chromatography (HIC) or anion exchange chromatography (A-IEX) and
(ii) the porous surfaces of component B are modified with functional moieties suitable for anion exchange chromatography (A-IEX) or hydrophobic interaction chromatography (HIC) whereby
(iii) the porous surfaces of components A and B are not modified with the moieties of (i) or (ii) simultaneously.

21. A chromatographic device having a porous self-supporting structure comprising at least two porous components A and B and the porous component B embraces the porous component A, wherein surfaces of pores of the at least two porous components A and B are provided with functional moieties suitable for chromatography of substances passing the pores and the pores of the porous components comprise a uniform multimodal pore size distribution through the entire polymeric structure, **characterized in that**
(i) the porous surfaces of component A are modified with functional moieties suitable for hydrophobic interaction chromatography (HIC) and
(ii) the porous surfaces of component B are modified with functional moieties suitable for anion exchange chromatography (A-IEX).

22. Use of a chromatographic device having a porous self-supporting structure comprising at least two porous components A and B and the porous component B embraces the porous component A, wherein surfaces of pores of the at least two porous components A and B are provided with functional moieties suitable for chromatography of substances passing the pores and the pores of the porous components comprise a uniform multimodal pore size distribution through the entire polymeric structure, wherein
(i) the porous surfaces of component A are modified with functional moieties suitable for hydrophobic interaction chromatography (HIC) or anion exchange chromatography (A-IEX) and
(ii) the porous surfaces of component B are modified with functional moieties suitable for anion exchange chromatography (A-IEX) or hydrophobic interaction chromatography (HIC) whereby
(iii) the porous surfaces of components A and B are not modified with the moieties of (i) or (ii) simultaneously,
for isolating and purifying a polynucleotide of interest on a manufacturing scale.

23. The use according to claim 22 for isolating and purifying plasmid DNA.
